# EUROPEAN PATENT SPECIFICATION

(11) **EP 0 742 787 B1**
(45) Date of publication and mention of the grant of the patent: **20.10.1999**
(21) Application number: 95910876.2
(22) Date of filing: 23.01.1995
(51) Int. Cl.: C07C 11/113, C07C 53/128

(54) **Process for preparing 2-ethyl-2-methylbutanoic acid from 3-methyl-2-pentene**
Verfahren zur Herstellung von 2-Ethyl-2-Methyl-Buttersäure aus 3-Methyl-2-Penten
Procédé pour la préparation de l'acide 2-éthyl-2-méthyl butyrique à partir du 3-méthyl-2-pentène

(30) Priority: 31.01.1994 US 189378
(43) Date of publication of application: 20.11.1996
(73) Proprietor: EXXON CHEMICAL PATENTS INC., Baytown, TX 77520-5200 (US)
(72) Inventor: HENDRIKSEN, Dan, E., Kingwood, TX 77345 (US); MCGLAMERY, Gerald, G., Baton Rouge, LA 70808 (US); KEENAN, Michael, J., Baton Rouge, LA 70817 (US); PETE, Derrick, D., Los Angeles, CA 90066 (US)
(74) Representative: Dew, Melvyn John
(86) International application number: US9500890
(87) International publication number: WO9520557

(56) References cited:
- EP-A- 0 356 518
- US-A- 4 691 073
- US-A- 5 254 785
- DATABASE WPI Section Ch, Week 4085 Derwent Publications Ltd., London, GB; Class E17, AN 85-247695 & SU,A,1 145 013 (PISMAN I I) , 15 March 1985 cited in the application

## Description

The present invention relates generally to a method for producing 3-methyl-2-pentene via the trimerization of ethylene to yield a C₆ product which contains 2-ethyl-1-butene, followed by etherification of 2-ethyl-1-butene in the C₆ product, separation of the ether and decomposition of the ether to 3-methyl-2-pentene. This process is capable of producing 3-methyl-2-pentene, substantially free of all other C₆ olefins, except 2-ethyl-1-butene, which is useful for the preparation of 2-ethyl-2-methylbutanoic acid, a high-value neoacid.

### BACKGROUND OF THE INVENTION

Researchers have been attempting for the past decade to develop an improved synthetic route to 2-ethyl-2-methylbutanoic acid (EMBA). The problem has been to develop an economically acceptable method for producing the olefin precursor, i.e., 3-methyl-2-pentene (3M2P) and/or 2-ethyl-1-butene (2E1B).

These olefin precursors are useful for the preparation of EMBA. However, the presence of even a few percent of any other C₆ olefin during preparation of this neoacid results in an unacceptable mixture of neoacids and neoacid esters that is exceedingly difficult to separate by distillation.

A number of refinery streams contain 3-methyl-2-pentene, but these streams contain many isomers of hexenes, and are not at all pure enough for the preparation of EMBA. 3-methyl-2-pentene may be produced from codimerizing ethylene with n-butenes over a supported sodium or potassium metal catalyst (see Soviet Union Patent No. 1,145,013 and Great Britain Patent No. 1,142,704), but these products also contain enough other isomers of hexene to make them unsuitable for the preparation of EMBA.

Mixed hexenes are produced as a by-product from the Alpbabutol™ process (a trademark of the Institut Francais du Petrole), which is used to make 1-butene.

A typical analysis of mixed hexenes discharged from the Alphabutol™ process is set forth below:

| **Component** | **Weight Percent** |
|---|---|
| 3-methyl-1-pentene | 26.0 |
| 1-hexene | 6.5 |
| 2-ethyl-1-butene | 65.0 |
| Others (C6 + C8) | 2.5 |

This process is described in the November, 1984, issue of "Hydrocarbon Processing", pp. 118-120. Significantly, the only isohexene contained in the C₆ by-product of the Alphabutol™ process is 2-ethyl-1-butene. There are no 2-methylpentenes or 2,3-dimethylbutenes, and essentially no methylcyclopentenes. Although the C₆ by-product of the Alphabutol™ process does contain significant amounts of EMBA precursor olefins, the presence of other C₆ olefins results in EMBA product that is not of sufficient purity. Conventional fractional distillation of the mixed hexenes yields streams enriched in desirable hexenes, but these enriched hexene streams still do not yield satisfactory EMBA product.

Processes to recover isobutylene and isoamylene from mixed streams of C₄ or C₅ olefins are known. These involve selectively forming an ether from the isobutylene or isoamylene, separating the ether, and decomposing it back to the C₄ or C₅ iso-olefin plus alcohol. [See US. Patent No. 4,691,073 (Michaelson), which issued on September 1, 1987).

There is only one isomer of isobutylene and only one skeletal isomer of isoamylene. There are four skeletal isomers of isohexene, i.e., 2-methylpentene, 2,3-dimethylbutene, 1-methyl cyclopentene, and 3-methyl-2-pentene or (2-ethyl-1-butene). All of these will react with an alcohol to form the respective ether.

The present inventors have developed a unique integrated process which combines the Alphatol™ process or like processes with an etherification/decomposition process which recovers a C₆ iso-olefin to produce a 3-methyl-2-pentene product which is substantially free of all other C₆ clefins, except 2-ethyl-1-butene.

The present invention also provides many additional advantages which shall become apparent as described below.

### SUMMARY OF THE INVENTION

According to the invention there is provided a method for producing 2-ethyl-2-methylbutanoic acid which comprises the steps of (i) producing 2-methyl-2-pentene which is substantially free of any other C₆ olefin, except 2-ethyl-1-butene, which comprises the steps of: (a) trimerizing an ethylene feedstream and/or co-dimerizing an ethylene and 1-butane feedstream in the presence of a titanium or zirconium based catalyst in order to form a hydrocarbon product comprising at least 2-ethyl-1-butene; (b) separating said hydrocarbon product from said catalyst; (c) etherifying said hydrocarbon product to form an ether-containing stream in the presence of either an acid resin catalyst or a zeolite catalyst; (d) separating an ether product from said ether-containing stream; (e) decomposing said ether product to form a 3-methyl-2-pentene stream in the presence of at least one catalyst selected from acid resin catalysts, zeolite catalysts, alumina catalysts and clay catalysts; and if) separating an alcohol and unreacted ether product from said 3-methyl-2-pentene stream to form a 3-methyl-2-pentene product; and (ii) mixing said 3-methyl-2-pentene product together with carbon monoxide, water, and a strong acid catalyst at a temperature of 25°C to 100°C and at a pressure in the range between 68.95 bar (1000 psig) to 206.85 bar (3000 psig) to form 2-ethyl-2-methylbutanoic acid.

Optionally, an additional step of separating a C₆ product which contains 2-ethyl-1-butene from the hydrocarbon product of step (b) and etherifying the C₆ product in step (c) instead of the hydrocarbon product may be employed in accordance with the present invention.

The present invention also pertains to a method for producing the intermediate material 3-methyl-2-pentene which is substantially free of any other C₆ olefin, except 2-ethyl-1-butene, which comprises the steps of: (a) trimerizing an ethylene feedstream and/or co-dimerizing an ethylene and 1-butene feedstream in the presence of a titanium or zirconium based catalyst in order to form a hydrocarbon product comprising at least 2-ethyl-1-butene; (b) separating the hydrocarbon product from the catalyst; (c) etherifying the hydrocarbon product to form an ether-containing stream; (d) separating an ether product from the ether-containing stream; (e) decomposing the ether product to form a 3-methyl-2-pentene stream; and (f) separating an alcohol and unreacted ether product from the 3-methyl-2-pentene stream to form a 3-methyl-2-pentene product.

Trimerization and/or co-dimerization step (a) is preferably conducted at a temperature in the range between 0°C to 100°C, more preferably between 50°C to 60°C. Trimerization and/or co-dimerization step (a) is also preferably conducted at a pressure sufficient to maintain the ethylene or ethylene and 1-butene in the liquid phase, i.e., a pressure in the range between 0.2 bar and 30.4 bar (0.2 to 30 atm).

Catalyst separation step (b) is carried out in an adsorption column or by simply distilling the hydrocarbon product away from the essentially non-volatile catalyst components.

The optional hydrocarbon separation step is carried out in a fractionation device. The preferred fractionation device comprises a first distillation column which is capable of separating the unreacted ethylene from 1-butene and heavier olefins and a second distillation column which is capable of separating 1-butene from a C₆ and heavier olefins product. A more preferred fractionation device comprises a first distillation column which is capable of separating the unreacted ethylene from 1-butene and heavier olefins, a second distillation column which is capable of separating 1-butene from C₆ and heavier olefins, and a third distillation column which is capable of separating a C₆ product from C₈ and heavier olefins.

Etherification step (c) comprises the following steps: (i) supplying the hydrocarbon product and at least one alcohol, e.g., methanol, to a reactor to produce an etherification mixture; and (ii) catalytically reacting the etherification mixture in the reactor containing an etherification catalyst under reaction conditions which favor forming an ether-containing stream from the hydrocarbon product and alcohol.

Ether separation step (d) comprises the feeding of the ether-containing stream into a distillation column. It is preferable that the distillation column has a catalytic distillation reaction zone including an acid resin catalyst to improve conversion of olefin to ether. Otherwise, unreacted olefin and methanol can be recycled to the etherification reactor to improve conversion.

Decomposition step (e) comprises contacting of the ether product with at least one catalyst selected from the group consisting of: acid resin catalysts, zeolite catalysts, alumina catalysts, and clay catalysts, especially hydrofluoric acid treated attapulgite clay catalysts (sold by Engelhard as D-5206G).

Separation step (f) comprises the feeding of the 3-methyl-2-pentene stream to extraction and distillation columns wherein alcohol and unreacted ether product are separated from the 3-methyl-2-pentene stream.

Other and further objects, advantages and features of the present invention will be understood by reference to the following specification in conjunction with the annexed drawings, wherein like parts have been given like numbers.

### BRIEF DESCRIPTION OF THE DRAWINGS

Fig. 1 illustrates the process flow diagram for the synthesis of 3-methoxy-3-methylpentane according to the present invention;
Fig. 2 illustrates the process flow diagram for the recovery of 3-methyl-2-pentene from 3-methoxy-3-methylpentane according to the present invention; and
Fig. 3 is a block diagram which depicts both the synthesis of 3-methoxy-3-methylpentane and recovery of 3-methyl-2-pentene according to the present invention.

### DESCRIPTION OF THE PREFERRED EMHBODIMENTS

The present invention involves the unique integration of two processes to produce a 3-methyl-2-pentene product which is substantially free of all other olefins, especially C₆ olefins, except 2-ethyl-1-butene. The first process involves the preparation of an olefin stream containing 2-ethyl-1-butene, with no other C₆ iso-olefins such as 2-methylpentene. The second process involves the recovery of this 2-ethyl-1-butene from the olefin stream via etherification to 3-methoxy-3-methylpentane, separation of the ether, and decomposition of the ether back to 3-methyl-2-pentene.

The reactions to produce 2-ethyl-1-butene from ethylene, or ethylene and 1-butene, are set forth below in equations 1 and 2.

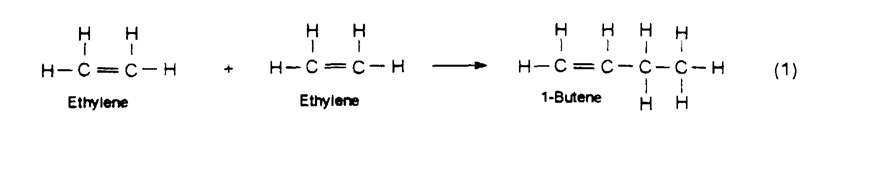

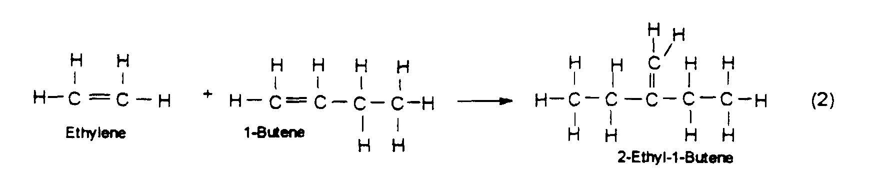

One process that uses these reactions was developed by Institut Francais du Petrole, and is known as the Alphabutol™ process. This process is used to prepare 1-butene by dimerizing ethylene. The C₆ olefins produced are considered by-products. The isohexene 2-ethyl-1-butene is a major C₆ product. By the nature of how they are produced, there are no other C₆ iso-olefins in this stream of C₆ olefins produced from ethylene.

The Alphabutol™ process uses a homogeneous catalyst which means that reactants, products and catalyst are all soluble in the same liquid phase. This catalyst is preferably a titanium based catalyst. Alternatively, heterogeneous catalysts such as a zirconium based catalyst can be used in this process.

The Alphabutol™ process is described in detail in Commereuc et al., "Dimerize Ethylene to Butene-1," Hydrocarbon Processing, November 1984, pp. 118-120. This is a simple process which is characterized by:
"(1) low operating temperature (50° to 60°C) requiring no heating medium for the reaction section; (2) low operating pressure sufficient only to maintain the reactants in the liquid phase; (3) no solvent requirement; and (4) no sophisticated materials requirement (only carbon steel is needed as a material of construction).
The process involves the feeding of polymer-grade ethylene, which is assumed available in the vapor phase, to a liquid phase reactor through a distributor. The dimerization reaction occurs in the liquid phase due to the specific catalyst which is continuously metered into the reactor solution. The heat of reaction is removed outside the reactor by a classical pump-around system, equipped with an air or water cooler. The product from the reactor is withdrawn from the bottom section as a liquid containing the spent catalyst.
The spent catalyst is then removed from the effluent and sent to disposal, e.g. incineration, while the hydrocarbons are routed to the fractionation section. In this section, the first tower separated the unconverted ethylene for recycle to the reactor, and a second column produces the pure butene-1 overhead and the oligomers (predominantly C₆ olefins) as bottoms."

Another process for synthesizing 1-butene by dimerization of ethylene and which also results in trimerization to 2-ethyl-1-butene is set forth in U.S. Patent No. 4,101,600 (Zhukov et al.), which issued on July 18, 1978, and which is incorporated herein by reference. Two patents which are related to the Alphabutol™ process and which are assigned to Institut Francais du Petrole are U.S. Patent Nos. 4,532,370 (Le Quan et al.), which issued on July 30, 1985, and 4,615,998 (Le Quan et al.), which issued on October 7, 1986. This later patent discloses a catalyst formed by reacting, in a hydrocarbon medium, a trialkyl-aluminum with a mixture of an alkyl titanate and an ether. Both of these patents are also incorporated herein by reference.

Processes to recover isobutylene and isoamylene from mixed streams of C₄ or C₅ olefins are known. These involve selectively forming an ether from the isobutylene or isoamylene, separating the ether, and decomposing it back to the C₄ or C₅ iso-olefin plus alcohol. (See U.S. Patent No. 4,691,073 (Michaelson), which issued on September 1, 1987).

The present inventors have utilized etherification to recover 2-ethyl-1-butene as the isomerized 3-methyl-2-pentene in accordance with the following reaction: 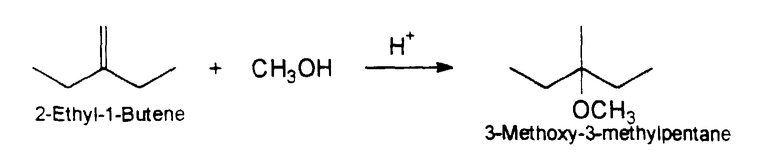 After this ether is separated by distillation from the unreacted hydrocarbons, 3-methyl-2-pentene is produced by decomposition of the ether in accordance with the following reaction: 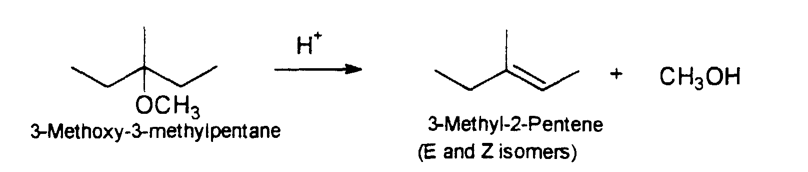 The 3-methyl-2-pentene is produced predominantly in the ether decomposition step because it is thermodynamically more stable than the 2-ethyl-1-butene.

The present invention involves the feeding of a mixture containing 2-ethyl-1-butene and methanol into the feed zone of a reactor (i.e., a fixed-bed guard reactor), and contacting the resultant mixture of 2-ethyl-1-butene and methanol with a fixed bed acidic cation exchange resin (e.g., Amberlyst® 15) in the reaction zone, thereby catalytically reacting the 2-ethyl-1-butene with the methanol under conditions which favor forming the resultant 3-methoxy-3-methylpentane.

Where the etherification step of the present invention is practiced in a catalytic distillation process, the catalytic material may be in any form which permits its incorporation into a distillation tower, such as a fixed bed, but may also be in a form which serves as a distillation packing, for example, rings, saddles, balls, irregular pieces, sheets, tubes, spirals, packed in bags, plated on grills or screens, and reticulated polymer foams.

Catalysts which have been found to be suitable for use in the etherification step of the present invention are resin catalysts such as cation exchange resin catalysts, acidic resin catalysts, macroreticular sulfonic acid cation exchange resin catalysts, and solid acid catalysts. Still others have used a zeolite as an etherification catalyst. Preferred catalysts for purposes of the present invention, however, are acid catalysts, such as acidic resin catalysts. A more preferred catalyst for purposes of the present invention is a macroreticular sulfonic acid cation exchange resin such as a sulfonated copolymer of polystyrene-divinylbenzene. Such catalysts include Amberlyst® 15 and 15C (marketed by Rohm and Haas), Lewatit SPC 118 and SPC 118 BG (marketed by Miles/Bayer), and Dowex M-31 and M-32 (marketed by the Dow Chemical Co.). A special version of this type of catalyst, i.e., Dowex DR-2040 (marketed by the Dow Chemical Co., is used specifically for reactive distillation.

The catalyst for the ether decomposition can be a solid acid such as an acid cation exchange resin, a clay, particularly hydrofluoric acid-treated attapulgite clay (sold by Engelhard as D-5206G), a zeolite compound or alumina. The zeolite catalyst is preferably a calcium Y zeolite as set forth in copending U.S. Patent Application, Serial No. 07/895,958.

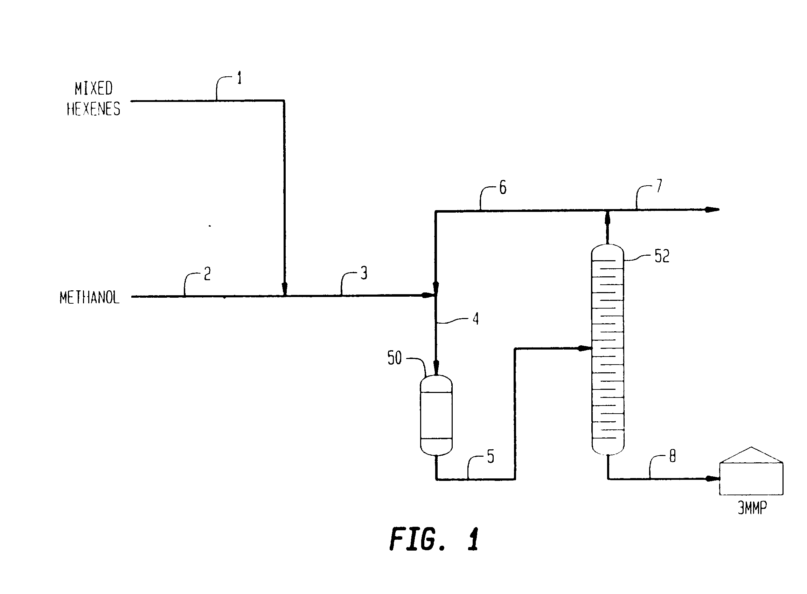

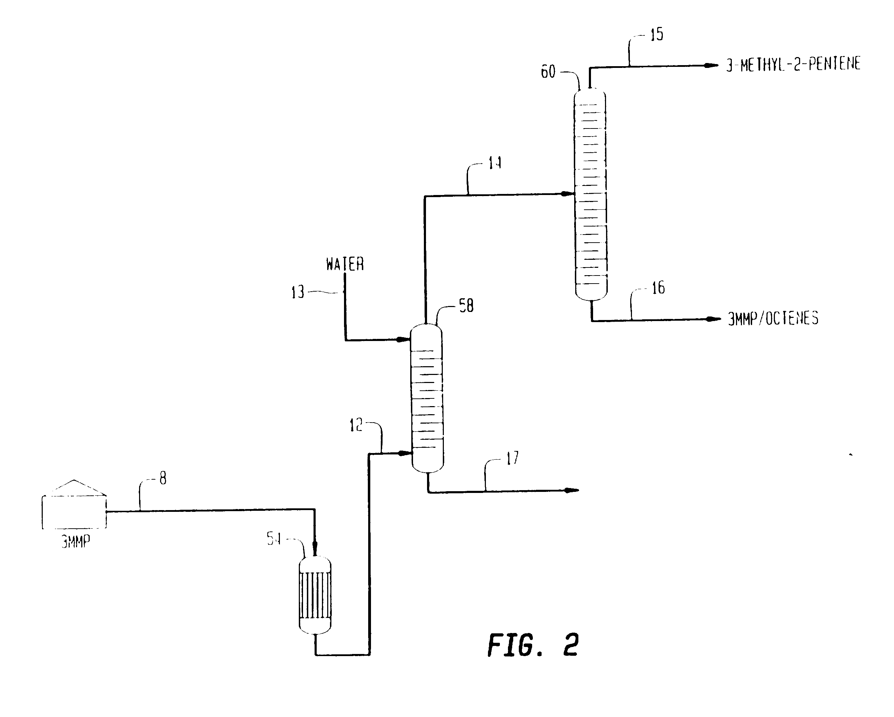

Referring now to Figs. 1 and 2, a schematic system is shown which can be used to recover 3-methyl-2-pentene.

A feedstream 1 of mixed hexenes produced from the Alphabutol™ process or its equivalent is mixed with a feedstream 2 of methanol. The mixture of hexenes and methanol are sent to etherification reactor 50 via conduits 3 and 4. The etherification reactor 50 is provided with an acidic resin catalyst, such as Amberlyst® 15 and 15C, Lewatit SPC 118 and SPC 118 BG, and Dowex M-31 and M-32, and is heated to an appropriate temperature (i.e., preferably 72°C to 78°C (130-140°F)) and maintained at a pressure sufficient to keep the reactants in the liquid phase. The ether-containing stream 5 leaving reactor 50 is composed of 3-methoxy-3-methylpentane (3MMP), unreacted hydrocarbons and methanol (CH₃OH). Ether-containing stream 5 is then fed to a distillation column 52. The overhead stream from distillation column 52 is composed of a raffinate which is depleted in 2-ethyl-1-butene. A portion of the raffinate may optionally be recycled to reactor 50 via conduit 6 to improve conversion. The raffinate is passed through methanol removal and final clean-up procedures via conduit 7. The bottoms stream 8 from distillation tower 52 contains 3MMP in admixture with hydrocarbons boiling higher than hexenes (if present). This stream 8 is passed to a decomposition reactor 54 wherein it is contacted with either an acidic resin catalyst, a clay catalyst, a zeolite catalyst, or an alumina catalyst at a preferred temperature of about 325°F (180°C) and a pressure in the range between 10 to 30 psig (1.7027 x 10⁵ N/m² to 5.1082 x 10⁵ N/m²) to form a 3-methyl-2-pentene stream. This 3-methyl-2-pentene stream is then sent via conduit 12 from decomposition reactor 54 to countercurrent extraction means 58, wherein water is added via conduit 13. The methanol and water are removed as bottoms via conduit 17. 3-Methyl-2-pentene, residual 3MMP ether, and heavy olefins (if present) are taken overhead via conduit 14 and sent to a distillation column 60, wherein 3-methyl-2-pentene is separated from the residual 3MMP and heavy olefins.

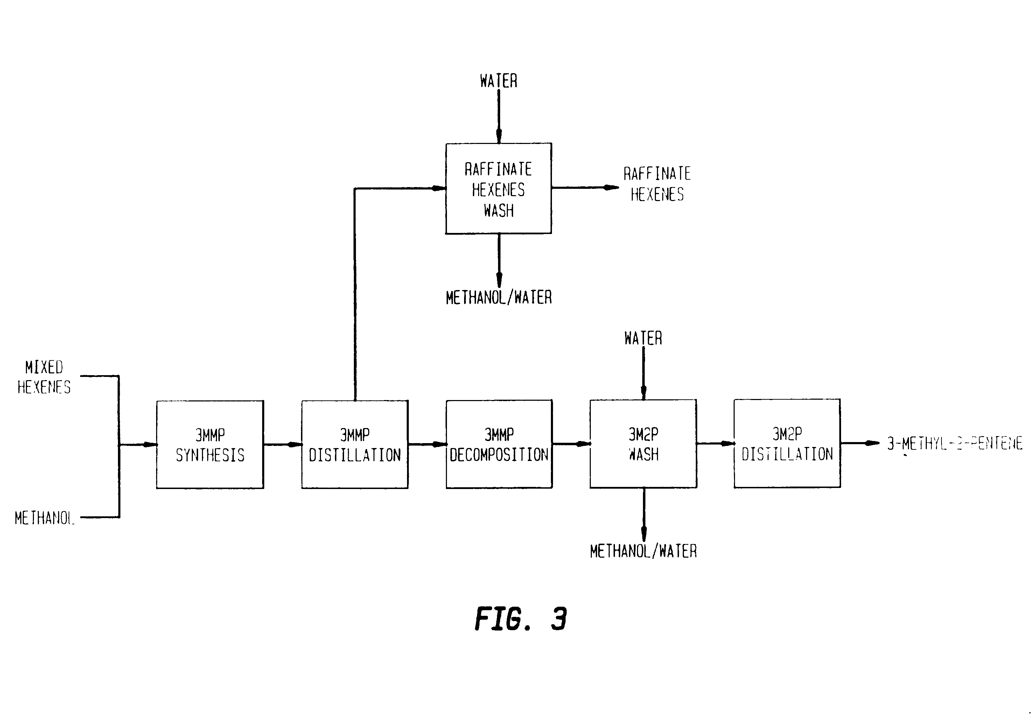

A block diagram for recovering 3-methyl-2-pentene from mixed hexenes and methanol according to the present invention is shown in Fig. 3:

The 3-methyl-2-pentene compound produced in accordance with the above method steps is substantially free of all other C6 olefins, except 2-ethyl-1-butene and is particularly useful in the preparation of 2-ethyl-2-methylbutanoic acid (EMBA). The 2-ethyl-2-methylbutanoic acid is a known commodity useful as an intermediate in the preparation of various herbicides. European Patent Specification No. 81304225.6 discloses herbicides which may be prepared from 2-ethyl-2-methylbutanoic acid.

2-Ethyl-2-methylbutanoic acid is preferably synthesized by reacting 3-methyl-2-pentene, 2-ethyl-1-butene, or a mixture thereof with carbon monoxide in the presence of an acid catalyst, followed by addition of water to the catalyzed product. This synthesis process provides an unusually high yield of the 2-ethyl-2-methylbutanoic acid product, especially when 3-methyl-2-pentene is carbonylated in the presence of an acid catalyst, preferably a strong acid catalyst, followed by reaction with water. Furthermore, only one C₇ by-product, i.e., 2,2-dimethylpentanoic acid, is formed in any significant amount (i.e., about 3-4 weight %) and conversion of the olefins is essentially quantitative.

Small portions of non-C₇ by-product acids are separable by distillation. Separation of C₇ acid isomers is difficult and typically requires superfractionation. Separation of hexyl alcohol esters of C₇ acids is also difficult and typically requires superfractionation. The importance of the invention is in providing 2-ethyl-2-methylbutanoic acid as the predominant C₇ isomer with little or no contamination by C₁₃ esters.

The process for synthesizing 2-ethyl-2-methylbutanoic acid is preferably carried out in a closed reactor under a pressure of carbon monoxide and preferably at elevated temperature. Any pressure and temperature may be used so long as the concentration of carbon monoxide present and the temperature of the reaction is sufficient to bring about conversion of the olefin reactant in the feedstream to the desired product, i.e., 2-ethyl-2-methylbutanoic acid. The temperature may range from a low temperature where the reaction will progress very slowly up to a high temperature wherein either the catalyst, materials, reactants, or products are destroyed by decomposition or other reaction. Suitable reaction temperatures range upwardly from at least about 25°C. For reactions with most of the acids which are effective as catalysts in the synthesis of 2-ethyl-2-methylbutanoic acid and for most carbon monoxide partial pressures, the reaction should generally be carried out at temperatures no higher than 72-82°C. This temperature range usually prevents decomposition of the catalyst material.

The synthesis of 2-ethyl-2-methylbutanoic acid may be carried out over a broad range of pressures so long as the desired end product is obtained from an olefin reactant of the feed. The range of pressures for the synthesis may vary broadly so long as the carbon monoxide is made available to the olefin reaction for formation of the desired product in the closed reactor.

An autoclave type reactor is suitable for preparation of the 2-ethyl-2-methylbutanoic acid. Mixtures of gases may be used to pressurize the reactor so long as a significant portion of the overall gas pressure is attributable to the carbon monoxide reactant. Gases which would interfere with or compete with the carbonylation should be avoided. Preferably, the reaction gas is at least about 50 weight percent carbon monoxide, more preferably 80-90 weight percent carbon monoxide and most preferably substantially entirely (100%) carbon monoxide. Suitable reaction pressures will range upwardly from 500 psig (3.549 x 10⁶ N/m²) carbon monoxide partial pressure to an upper limit which is generally determined by the capabilities of the reaction vessel. Preferably, a stainless steel or a similar vessel is used to avoid problems with the acid catalyst, and pressures available for operation of the process will then depend upon the capabilities of the reactor. A good operating range for the synthesis of 2-ethyl-2-methylbutanoic acid is above 1,200 psig (8.375 x 10⁶ N/m²) carbon monoxide partial pressure. A desirable operating pressure is above 3,000 psig (2.0938 x 10⁷ N/m²) if the equipment is suitable for such high pressures.

Diluents including inert paraffinic solvents such as heptane or hexane may be used and may in fact improve selectivity somewhat. However, they are not required according to the invention. Diluent or solvent portions of the acid catalyst may be used and reused for the reaction.

The preferred synthesis involves the reaction of a 3-methyl-2-pentene olefin feed with carbon monoxide in the presence of an acid catalyst, followed by contact, reaction, or "quenching" with water to provide the desired 2-ethyl-2-methylbutanoic acid in high yield. Those acids generally classified as strong acids are preferred, strong inorganic acids being more preferred catalysts. Use of concentrated acids is also preferred. Suitable acids for reaction with the olefins include sulfuric acid, phosphoric acid, hydrochloric acid, boron trifluoride dihydrate, and other acids in this family or those of another type which bring about the conversion of the C₆ branched olefin to the 2-ethyl-2-methylbutanoic acid.

After the C₆ branched olefin in the reaction feed is contacted with an acid and carbonylated, the reaction product is quenched or otherwise contacted with water to form the 2-ethyl-2-methylbutanoic acid. It has been found that phosphoric acid has the best reactivity with the C₆ branched olefins, but phosphoric acid, like sulfuric acid, has a high corrosivity problem. Accordingly, a good compromise acid, where required for the synthesis of 2-ethyl-2-methylbutanoic acid is boron trifluoride dihydrate (BF₃•2H₂O).

In the water addition step, solutions of water may also be used so long as the other ingredients in the solution do not interfere with the recovery of the 2-ethyl-2-methylbutanoic acid.

The preferred neoacid synthesis process comprises reacting one mole part of 3-methyl-2-pentene and/or 2-ethyl-1-butene at an effective temperature and carbon monoxide pressure in the presence of an acid followed by contacting the reaction mixture with water, preferably in excess, so as to form the desired 2-ethyl-2-methylbutanoic acid product in high yield. The carbon monoxide pressure in the reactor may be started at a sufficiently high level or with a sufficiently large over-pressure space that, during the course of the reaction, the amount of carbon monoxide is not significantly depleted. Alternatively, the carbon monoxide pressure may be maintained at a desired level or otherwise varied by supplying additional carbon monoxide or carbon monoxide-containing gas mixture to the reactor during the course of the reaction. Preferably, the carbon monoxide is bubbled through the bottom of the reactant liquid olefin mixture.

The amount of acid used depends upon the strength of the acid, i.e., the type of acid used and the concentration of the acid. Generally, for each mole part of reactant olefin(s) at least about 1.0 mole part of acid is used. The acid catalyst is preferably used in excess since it is recovered. 1.0 to 2.0 mole parts is usable, preferably 1.0 to 1.5, more preferably 1.0 to 1.1 mole parts. Larger amounts of acid may be used as diluent. The more limited range for the acid catalyst to conserve resources on a commercial scale is 1 to 1.1 mole parts of acid per mole part of reactant olefin. Alternatively, large acid/olefin ratio reactions may be conducted and the acid recycled.

### EXAMPLE 1

The following example illustrates a laboratory method for recovery of 3-methyl-2-pentene (containing some 2-ethyl-1 butene).

The solid acid catalyst used in both ether synthesis and decomposition is Amberlyst® 15, a macroreticular ion exchange resin in the acid form. The Amberlyst® 15 was washed with distilled water until the washes were colorless, and then dried in an oven overnight at 110°C. A mixture of C₆ olefins obtained as a by-product from the Alphabutol™ process was analyzed by gas chromatography, and a 10% molar excess of methanol over the analyzed 2-ethyl-1-butene content was added. Dry Amberlyst® 15 was added to this mixture and the slurry was stirred at room temperature until about half of the 2-ethyl-1-butene was converted to an ether, i.e., 3-methoxy-3-methylpentane. (This conversion is limited by equilibrium). The solid acid resin was removed by filtration and the unreacted methanol was removed by washing four times with distilled water. The resulting mixture of ether and hydrocarbon was distilled, and a heartcut of ether was obtained. The isolated ether was then decomposed back to olefin and methanol. This was done by adding dry Amberlyst® 15 to the ether, heating, and distilling off the olefin and the methanol as they were formed. The collected material was washed several times with distilled water to remove the methanol, and was then distilled one more time (from added xylenes to avoid distillation to dryness). The product purity was 99.9% of the desired two olefins, i.e., 5.01% 2-ethyl-1-butene and 94.90% 3-methyl-2-pentene (two isomers).

### EXAMPLE 2

A mixture of C₆ olefins (including some heavier olefins) obtained as a by-product of the Alphabutol™ process was mixed with technical grade methanol and fed to a series of three tubular reactors. Each reactor was nominally 1 inch (2.54 cm) in diameter and 20 feet (6.096 m) in length, and each was packed with Dowex® M-31 ion exchange resin. The reactors were maintained at temperatures in the range between 110°F (61.1°C) to 150°F (83.3°C). Mass flow rates to the reactor train were varied from 33 to 39 lbₘ/h (14.97 to 17.69 kgₘ/h). The molar ratio of methanol to 2-ethyl-1-butene was varied from 1.1 to 1.3. Reactor train outlet pressure was maintained at 20 psig (2.392 x 10⁵ N/m²).

The effluent from the synthesis reactors was then distilled in a 6 inch (15.24 cm) diameter fractionation column containing 50 feet (15.24 m) of packing. Methanol and unreacted hexenes were taken as an overhead stream, while 3-methoxy-3-methylpentane and heavy olefins were taken as a bottom stream.

The 3MMP and heavy olefins stream was then fed to a 1 inch (2.54 cm) diameter, 20 feet (6.096 m) long, tubular reactor. The reactor was packed with either granular, hydrofluoric acid-treated clay, or with calcium Y zeolite extrudate. For the HF-treated clay, reactor temperatures were varied between 300 and 375°F (167 to 208°C), and mass flow rates were varied from 9.7 to 11.7 1bₘ/h (4.4 to 5.3 kg/h). For the calcium Y zeolite, reactor temperatures were varied between 300 and 350°F (166.7 to 194.4°C), and mass flow rates were varied from 13.8 to 19.3 1bₘ/h (6.26 to 8.75 kg/h). Reactor outlet pressure was maintained at 5 to 10 psig (1.358 x 10⁵ to 1.703 x 10⁵ N/m²).

The resulting product was washed in a four-stage, cross-current, mixer-settler extraction with water used to remove methanol. The product was then distilled in a 3 inch (7.62 cm) diameter fractionation column containing 44 feet (13.4 m) of packing. The 3-methyl-2-pentene and 2-ethyl-1-butene were taken as an overhead stream, while the unreacted 3MMP and heavy olefins were taken as a bottom stream.

The final product, when analyzed by gas chromatography, had the composition set forth below in Table 1:

**Table 1**

| **(Composition of 3-Methyl-2-Pentene Final Product)** | |
|---|---|
| **Component** | **Weight Percent** |
| 2-ethyl-1-butene | 10.30 |
| 3-methyl-2-pentene (E & Z) | 89.24 |
| other hydrocarbon | 0.46 |

### EXAMPLE 3

A sample of 3-methyl-2-pentene concentrate was prepared in accordance with the steps of Example 1. The composition of the material, as measured by gas chromatography, is shown in Table 2.

**Table 2**

| **(Composition of 3-Methyl-2-Pentene Concentrate)** | |
|---|---|
| **Component** | **Weight Percent** |
| 2-ethyl-1-butene | 5.01 |
| 3-methyl-2-pentene (E & Z) | 94.90 |
| other hydrocarbon | 0.09 |

A 400 mL quantity of the 3-methyl-2-pentene concentrate was added to a 1 liter autoclave reactor. The reactor agitator was started, and the reactor was pressurized to 1300 psig (9.0645 x 10⁶ N/m²) with carbon monoxide. Over a period of 160 minutes, 300 mL of BF₃•2H₂O were added to the reactor so that the final reactor pressure was 1500 psig (1.03 x 10⁷ N/m²). At the end of this period, the addition of BF₃•2H₂O was stopped, and the contents were allowed to mix for another 300 minutes. During the reaction, the reactor contents were maintained at a temperature of 52°C.

At the end of 300 minutes, the reactor contents were washed with water to remove the catalyst. The resulting product had the component make-up as set forth below in Table 3.

**Table 3**

| **(3-Methyl-2-Pentene Concentrate Carbonylation Reactor Product)** | |
|---|---|
| **Component** | **Weight Percent** |
| olefins | 2.96 |
| C5 acids | 0.87 |
| C6 acids | 3.74 |
| C7 esters | 0.00 |
| 2,2-dimethylpentanoic acid | 3.33 |
| 2,2,3-trimethylbutanoic acid | 0.08 |
| 2-ethyl-2-methylbutanoic acid | 79.89 |
| C8 acids | 3.86 |
| C9 acids | 2.10 |
| C10 acids | 0.70 |
| C13 acids | 2.48 |
| heavy acids | 0.00 |

The selectivity among the C₇ acid isomers is shown in Table 4 below.

**Table 4**

| **(C7 Acid Isomer Selectivity from 3M2P Concentrate)** | |
|---|---|
| **Component** | **Weight Percent** |
| 2,2-dimethylpentanoic acid | 4.00 |
| 2,2,3-trimethylbutanoic acid | 0.09 |
| 2-ethyl-2-methylbutanoic acid | 95.91 |

### COMPARATIVE EXAMPLE 4

This comparative example shows that when C6 olefins, other than 3M2P or 2E1B, are present, the quality of the neoacid product is unacceptable. A sample of 2-ethyl-1-butene concentrate was prepared by superfractionation from the 2-ethyl-1-butene product of the ethylene trimerization. The composition of the material, as measured by gas chromatography, is shown in Table 5 below.

**Table 5**

| **(Composition of 2-Ethyl-1-Butene Concentrate)** | |
|---|---|
| **Component** | **Weight Percent** |
| 3-methyl-1-pentene | 0.11 |
| 1-hexene | 8.73 |
| 1,4-hexadiene | 1.86 |
| 2-ethyl-1-butene | 87.08 |
| t-2-hexene | 0.72 |

A 400 mL quantity of the 2-ethyl-1-butene concentrate was added to a 1 liter autoclave reactor. The reactor agitator was started, and the reactor was pressurized to 1300 psig (9.0645 x 10⁶ N/m²) with carbon monoxide. Over a period of 160 minutes, 300 mL of BF₃•2H₂O were added to the reactor so that the final reactor pressure was 1500 psig (1.03 x 10⁷ N/m²). At the end of this period, the addition of BF₃•2H₂O was stopped, and the contents were allowed to mix for another 300 minutes. During the reaction, the reactor contents were maintained at a temperature of 52°C.

At the end of 300 minutes, the reactor contents were washed with water to remove the catalyst. The resulting product had the component make-up as set forth below in Table 6.

**Table 6**

| **(2-Ethyl-1-Butene Concentrate Carbonylation Reactor Product)** | |
|---|---|
| **Component** | **Weight Percent** |
| olefins | 5.05 |
| C5 acids | 1.01 |
| C6 acids | 3.85 |
| C7 esters | 4.05 |
| 2,2-dimethylpentanoic acid | 4.84 |
| 2,2,3-trimethylbutanoic acid | 0.26 |
| 2-ethyl-2-methylbutanoic acid | 46.10 |
| C8 acids | 6.74 |
| C9 acids | 2.89 |
| C10 acids | 3.16 |
| C13 acids | 15.58 |
| heavy acids | 6.47 |

The selectivity among the C₇ acid isomers is shown in Table 7 below.

**Table 7**

| **(C7 Acid Isomer Selectivity from 2-Ethyl-1-Butene Concentrate)** | |
|---|---|
| **Component** | **Weight Percent** |
| 2,2-dimethylpentanoic acid | 9.45 |
| 2,2,3-trimethylbutanoic acid | 0.50 |
| 2-ethyl-2-methylbutanoic acid | 90.05 |

The 2-ethyl-2-methylbutanoic acid sample produced in the present example, when distilled to remove acids of other carbon numbers, would not meet commercial specifications.

While we have shown and described several embodiments in accordance with our invention, it is to be clearly understood that the same are susceptible to numerous changes apparent to one skilled in the art. Therefore, we do not wish to be limited to the details shown and described but intend to show all changes and modifications which come within the scope of the appended claims.

## Claims

1. A method for producing 2-ethyl-2-methylbutanoic acid which comprises the steps of:
(i) producing 3-methyl-2-gentene which is substantially free of any other C₆ olefin, except 2-ethyl-1-butane, which comprises the steps of:
(a) trimerizing an ethylene feedstream and/or co-dimerizing an ethylene and 1-butene feedstream in the presence of a titanium or zirconium based catalyst in order to form a hydrocarbon product comprising at least 2-ethyl-1-butene;
(b) separating said hydrocarbon product from said catalyst;
(c) etherifying said hydrocarbon product to form an ether-containing stream in the presence of either an acid resin catalyst or a zeolite catalyst;
(d) separating an ether product from said ether-containing stream;
(e) decomposing said ether product to form a 3-methyl-2-pentene stream in the presence of at least one catalyst selected from acid resin catalysts, zeolite catalysts, alumina catalysts and clay catalysts; and
(f) separating an alcohol and unreacted ether product form said 3-methyl-2-pentene stream to form a 3-methyl-2-pentene product; and
(ii) mixing said 3-methyl-2-pentene product together with carbon monoxide, water, and strong acid catalyst at a temperature of 25°C to 100°C and at a pressure in the range between 68.95 bar (1000 psig) to 206.85 bar (3000 psig) to form 2-ethyl-2-methylbutanoic acid.

2. The method according to claim 1 further comprising the step of separating a C6 product, with or without heavier olefins and which contains 2-ethyl-1-butene, from said hydrocarbon product of step (b) and etherifying said C6 product in step (c) instead of said hydrocarbon product.

3. A method for producing 3-methyl-2-pentene which is substantially free of any other C6 olefin, except 2-ethyl-1-butene suitable for use in the method of claim 1 which comprises the steps of:
(a) trimerizing an ethylene feedstream and/or co-dimerizing an ethylene and 1-butene feedstream in the presence of a titanium or zirconium based catalyst in order to form a hydrocarbon product comprising at least 2-ethyl-1-butene;
(b) separating said hydrocarbon product from said catalyst;
(c) etherifying said hydrocarbon product to form an ether-containing stream in the presence of either an acid resin catalyst or a zeolite catalyst;
(d) separating an ether product from said ether-containing stream;
(e) decomposing said ether product to form a 3-methyl-2-pentene stream in the presence of at least one catalyst selected from acid resin catalysts, zeolite catalysts, alumina catalysts and clay catalysts; and
(f) separating an alcohol and unreacted ether product from said 3-methyl-2-pentene stream to form a 3-methyl-2-pentene product.

4. The method according to claim 3 further comprising the step of separating a C6 product, with or without heavier olefins and which contains 2-ethyl-1-butene, from said hydrocarbon product of step (b) and etherifying said C6 product in step (c) instead of said hydrocarbon product.

5. The method according to either of claims 3 or 4 wherein said trimerization and/or co-dimerization step (a) is conducted at a temperature in the range between 0°C to 100°C.

6. The method according to claim 5 wherein said trimerization and/or co-dimerization step (a) is conducted at a temperature in the range between 50°C to 60°C.

7. The method according to any of claims 3 to 6 wherein said trimerization and/or co-dimerization step (a) is conducted at a pressure sufficient to maintain said ethylene and 1-butene in the liquid phase.

8. The method according to claim 7 wherein said pressure is in t he range between 0.2 to 30 atm.

9. The method according to any of claims 3 to 8 wherein the catalyst used in step (a) is obtained by reacting a mixture of alkyl titanate and ether with an alkylaluminum compound.

10. The method according to any of claims 3 to 9 wherein said separation step (b) is carried out in an adsorption column or by distilling said hydrocarbon product away from the essentially non-volatile catalyst components.

11. The method according to claim 4 wherein the separation of said C6 product comprising 2-ethyl-1-butene from said hydrocarbon product is carried out in a fractionation device.

12. The method according to claim 11 wherein said fractionation device comprises a first column which is capable of separating the unreacted ethylene from 1-butene and heavier olefins and a second column which is capable of separating 1-butene from a C6 product and heavier olefins product.

13. The method according to either of claims 11 or 12 wherein said fractionation device comprises a first column which is capable of separating the unreacted ethylene from 1-butene and heavier olefins, a second column which is capable of separating 1-butene from C6 and heavier olefins and a third column which is capable of separating a C6 product from C8 and heavier olefins.

14. The method according to any of claims 3 to 13 wherein said etherification step (c) comprises the following steps:
(i) supplying said hydrocarbon product and at least one alcohol to a reactor to produce an etherification mixture; and
(ii)catalytically reacting said etherification mixture in said reactor containing an etherification catalyst under reaction conditions which favour forming an ether-containing stream from said hydrocarbon product and said alcohol.

15. The method according to claim 14 wherein said etherification catalyst is an acid resin catalyst selected from the group consisting of: cation exchange resin catalysts and macroreticular sulfonic acid cation exchange resin catalysts.

16. The method according to any of claims 3 to 15 wherein said separation step (d) comprises the feeding of said ether-containing stream into a distillation column to separate said ether product from said ether-containing stream.

17. The method according to claim 14 wherein said alcohol i s methanol and said ether product is 3-methoxy-3-methylpentane.

18. The method according to claim 17 wherein said decomposition step (e) is capable of decomposing said 3-methoxy-3-methylpentane to form at least 3-methyl-2-pentene and methanol.

19. The method according to any of claims 3 to 18 wherein said separation step (f) comprises the extraction with water for the removal of alcohol(s) from the 3-methyl-2-pentene stream, and distillation of the alcohol depleted 3-methyl-2-pentene from said 3-methyl-2-pentene stream to form said 3-methyl-2-pentene product.

## Patentansprüche

1. Verfahren zur Herstellung von 2-Ethyl-2-methylbutansäure, bei dem
(i) 3-Methyl-2-penten hergestellt wird, das im wesentlichen frei von jeglichem anderen C₆-Olefin außer 2-Ethyl-1-buten ist, wobei
(a) in Gegenwart von Katalysator auf Titan- oder Zirconiumbasis ein Ethyleneinsatzmaterialstrom trimerisiert wird und/oder ein Ethylen- und 1-Buteneinsatzmaterialstrom codimerisiert wird, um ein Kohlenwasserstoffprodukt zu bilden, das mindestens 2-Ethyl-1-buten umfaßt,
(b) das Kohlenwasserstoffprodukt von dem Katalysator abgetrennt wird,
(c) das Kohlenwasserstoffprodukt unter Bildung eines etherhaltigen Stroms in Gegenwart von entweder einem sauren Harzkatalysator oder einem Zeolithkatalysator verethert wird;
(d) ein Etherprodukt aus dem etherhaltigen Strom abgetrennt wird,
(e) das Etherprodukt in Gegenwart von mindestens einem Katalysator ausgewählt aus sauren Harzkatalysatoren, Zeolithkatalysatoren, Aluminiumoxidkatalysatoren und Tonkatalysatoren unter Bildung eines 3-Methyl-2-pentenstroms zersetzt wird, und
(f) ein Alkohol und nicht-umgesetztes Etherprodukt aus dem 3-Methyl-2-pentenstrom abgetrennt wird, um ein 3-Methyl-2-pentenprodukt zu bilden, und
(ii) das 3-Methyl-2-pentenprodukt bei einer Temperatur von 25°C bis 100°C und einem Druck im Bereich von 68,95 bar (1000 psig) bis 206,85 bar (3000 psig) zusammen mit Kohlenmonoxid, Wasser und einer starken Säure als Katalysator gemischt wird, um 2-Ethyl-2-methylbutansäure zu bilden.

2. Verfahren nach Anspruch 1, bei dem in einer weiteren Stufe ein C₆-Produkt mit oder ohne schwereren Olefinen, das 2-Ethyl-1-buten enthält, von dem Kohlenwasserstoffprodukt aus Stufe (b) abgetrennt wird und das C₆-Produkt in Stufe (c) anstelle des Kohlenwasserstoffprodukts verethert wird.

3. Verfahren zur Herstellung von 3-Methyl-2-penten, das im wesentlichen frei von jedem anderen C₆-Olefin außer 2-Ethyl-1-buten ist und zur Verwendung in dem Verfahren gemäß Anspruch 1 geeignet ist, bei dem
(a) in Gegenwart von Katalysator auf Titan- oder Zirconiumbasis ein Ethyleneinsatzmaterialstrom trimerisiert wird und/oder ein Ethylen- und 1-Buteneinsatzmaterialstrom codimerisiert werden, um ein Kohlenwasserstoffprodukt zu bilden, das mindestens 2-Ethyl-1-buten umfaßt,
(b) das Kohlenwasserstoffprodukt von dem Katalysator abgetrennt wird,
(c) das Kohlenwasserstoffprodukt unter Bildung eines etherhaltigen Stroms in Gegenwart von entweder einem sauren Harzkatalysator oder einem Zeolithkatalysator verethert wird;
(d) ein Etherprodukt aus dem etherhaltigen Strom abgetrennt wird,
(e) das Etherprodukt in Gegenwart von mindestens einem Katalysator ausgewählt aus sauren Harzkatalysatoren, Zeolithkatalysatoren, Aluminiumoxidkatalysatoren und Tonkatalysatoren unter Bildung eines 3-Methyl-2-pentenstroms zersetzt wird, und
(f) ein Alkohol und nicht-umgesetztes Etherprodukt aus dem 3-Methyl-2-pentenstrom abgetrennt wird, um ein 3-Methyl-2-pentenprodukt zu bilden.

4. Verfahren nach Anspruch 3, bei dem in einer weiteren Stufe ein C₆-Produkt mit oder ohne schwerere Olefine, das 2-Ethyl-1-buten enthält, von dem Kohlenwasserstoffprodukt aus Stufe (b) abgetrennt wird und das C₆-Produkt in Stufe (c) anstelle des Kohlenwasserstoffprodukts verethert wird.

5. Verfahren nach einem der Ansprüche 3 oder 4, bei dem die Trimerisierungs- und/oder Codimerisierungsstufe (a) bei einer Temperatur im Bereich von 0°C bis 100°C durchgeführt wird.

6. Verfahren nach Anspruch 5, bei dem die Trimerisierungs- und/oder Codimerisierungsstufe (a) bei einer Temperatur im Bereich von 50°C bis 60°C durchgeführt wird.

7. Verfahren nach einem der Ansprüche 3 bis 6, bei dem die Trimerisierungs- und/oder Codimerisierungsstufe (a) bei einem ausreichenden Druck durchgeführt wird, um das Ethylen und 1-Buten in der flüssigen Phase zu halten.

8. Verfahren nach Anspruch 7, bei dem der Druck im Bereich von 0,2 bis 30 atm liegt.

9. Verfahren nach einem der Ansprüche 3 bis 8, bei dem der in Stufe (a) verwendete Katalysator erhalten wird, indem eine Mischung aus Alkyltitanat und Ether mit einer Alkylaluminiumverbindung umgesetzt wird.

10. Verfahren nach einem der Ansprüche 3 bis 9, bei dem die Abtrennungsstufe (b) in einer Adsorptionssäule durchgeführt wird oder indem das Kohlenwasserstoffprodukt von den im wesentlichen nicht-flüchtigen Katalysatorkomponenten abdestilliert wird.

11. Verfahren nach Anspruch 4, bei dem die Abtrennung des 2-Ethyl-1-buten umfassenden C₆-Produkts von dem Kohlenwasserstoffprodukt in einer Fraktioniervorrichtung durchgeführt wird.

12. Verfahren nach Anspruch 11, bei dem die Fraktioniervorrichtung eine erste Kolonne, die das nicht umgesetzte Ethylen von 1-Buten und schwereren Olefinen abtrennen kann, und eine zweite Kolonne umfaßt, die das 1-Buten von C₆-Produkt und schweren Olefinprodukten abtrennen kann.

13. Verfahren nach einem der Ansprüche 11 oder 12, bei dem die Fraktioniervorrichtung eine erste Kolonne, die das nicht umgesetzte Ethylen von 1-Buten und schwereren Olefinen abtrennen kann, eine zweite Kolonne, die das 1-Buten von C₆-Produkt und schweren Olefinprodukten abtrennen kann, und eine dritte Kolonne umfaßt, die C₆-Produkt von C₈ und schwereren Olefinen abtrennen kann.

14. Verfahren nach einem der Ansprüche 3 bis 13, bei dem die Veretherungsstufe (c) die folgenden Stufen umfaßt:
(i) Zufuhr des Kohlenwasserstoffprodukts und mindestens eines Alkohols zu einem Reaktor, um eine Veretherungsmischung herzustellen, und
(ii) katalytische Umsetzung dieser Veretherungsmischung in dem Reaktor, der einen Veretherungskatalysator enthält, unter Reaktionsbedingungen, die die Bildung eines etherhaltigen Stroms aus dem Kohlenwasserstoffprodukt und dem Alkohol begünstigen.

15. Verfahren nach Anspruch 14, bei dem der Veretherungskatalysator ein saurer Harzkatalysator ausgewählt aus der Gruppe bestehend aus Kationaustauscherharzkatalysatoren und makroretikulären Sulfonsäure-Kationaustauschharzkatalysatoren ist.

16. Verfahren nach einem der Ansprüche 3 bis 15, bei dem in der Abtrennungsstufe (d) der etherhaltige Strom in eine Destillationskolonne eingespeist wird, um das Etherprodukt von dem etherhaltigen Strom abzutrennen.

17. Verfahren nach Anspruch 14, bei dem der Alkohol Methanol und das Etherprodukt 3-Methoxy-3-methylpentan ist.

18. Verfahren nach Anspruch 17, bei dem die Zersetzungsstufe (e) in der Lage ist, das 3-Methoxy-3-methylpentan unter Bildung von mindestens 3-Methyl-2-penten und Methanol zu zersetzen.

19. Verfahren nach einem der Ansprüche 3 bis 18, bei dem die Abtrennungsstufe (f) die Extraktion mit Wasser zur Entfernung von Alkohol(en) aus dem 3-Methyl-2-pentenstrom und Destillation des an Alkohol verarmten 3-Methyl-2-pentens von dem 3-Methyl-2-pentenstrom umfaßt, um das 3-Methyl-2-pentenprodukt zu bilden.

## Revendications

1. Procédé pour la production d'acide 2-éthyl-2-méthylbutanoïque, qui comprend les étapes suivantes :
(i) production de 3-méthyl-2-pentène qui est pratiquement dépourvu de toute autre oléfine en C_{6,} à l'exception du 2-éthyl-1-butène, qui comprend les étapes consistant :
(a) à trimériser un courant d'éthylène d'alimentation et/ou co-dimériser un courant d'éthylène et de 1-butène d'alimentation en présence d'un catalyseur à base de titane ou de zirconium afin de former un produit hydrocarboné comprenant au moins du 2-éthyl-1-butène ;
(b) à séparer ledit produit hydrocarboné dudit catalyseur ;
(c) à éthérifier ledit produit hydrocarboné pour former un courant contenant un éther en présence d'un catalyseur consistant en une résine acide ou d'un catalyseur zéolitique ;
(d) à séparer un produit consistant en un éther dudit courant contenant un éther ;
(e) à décomposer ledit produit consistant en un éther pour former un courant de 3-méthyl-2-pentène en présence d'au moins un catalyseur choisi entre des catalyseurs consistant en des résines acides, des catalyseurs zéolitiques, des catalyseurs à base d'alumine et des catalyseurs à base d'argiles ; et
(f) à séparer un alcool et le produit consistant en éther n'ayant pas réagi dudit courant de 3-méthyl-2-pentène pour former un produit consistant en 3-méthyl-2-pentène ; et
(ii) mélange dudit produit consistant en 3-méthyl-2-pentène avec du monoxyde de carbone, de l'eau et un catalyseur du type acide fort à une température comprise dans l'intervalle de 25°C à 100°C et sous une pression comprise dans l'intervalle de 68,95 bars (1000 psig) à 206,85 bars (3000 psig) pour former de l'acide 2-éthyl-2-méthylbutanoïque.

2. Procédé suivant la revendication 1, comprenant en outre l'étape de séparation d'un produit en C_{6,} avec ou sans des oléfines plus lourdes et qui contient du 2-éthyl-1-butène, dudit produit hydrocarboné de l'étape (b) et d'éthérification dudit produit en C₆ dans l'étape (c) au lieu dudit produit hydrocarboné.

3. Procédé pour la production de 3-méthyl-2-pentène qui est pratiquement dépourvu de toute autre oléfine en C_{6,} à l'exception du 2-éthyl-1-butène, convenable pour une utilisation dans le procédé suivant la revendication 1, qui comprend les étapes consistant :
(a) à trimériser un courant d'éthylène d'alimentation et/ou à co-dimériser un courant d'éthylène et de 1-butène d'alimentation en présence d'un catalyseur à base de titane ou de zirconium afin de former un produit hydrocarboné comprenant au moins du 2-éthyl-1-butène ;
(b) à séparer ledit produit hydrocarboné dudit catalyseur ;
(c) à éthérifier ledit produit hydrocarboné pour former un courant contenant un éther en présence d'un catalyseur consistant en une résine acide ou d'un catalyseur zéolitique ;
(d) à séparer un produit consistant en un éther dudit courant contenant un éther ;
(e) à décomposer ledit produit consistant en un éther pour former un courant de 3-méthyl-2-pentène en présence d'au moins un catalyseur choisi entre des catalyseurs consistant en résines acides, des catalyseurs zéolitiques, des catalyseurs à base d'alumine et des catalyseurs à base d'argiles ; et
(f) à séparer un alcool et le produit consistant en éther n'ayant pas réagi dudit courant de 3-méthyl-2-pentène pour former comme produit du 3-méthyl-2-pentène.

4. Procédé suivant la revendication 3, comprenant en outre l'étape consistant à séparer un produit en C₆, avec ou sans des oléfines plus lourdes et qui contient du 2-éthyl-1-butène, dudit produit hydrocarboné de l'étape (b) et à éthérifier ledit produit en C₆ dans l'étape (c) au lieu dudit produit hydrocarboné.

5. Procédé suivant la revendication 3 ou 4, dans lequel l'étape de trimérisation et/ou de co-dimérisation (a) est mise en oeuvre à une température comprise dans l'intervalle de 0°C à 100°C.

6. Procédé suivant la revendication 5, dans lequel l'étape de trimérisation et/ou de co-dimérisation (a) est mise en oeuvre à une température comprise dans l'intervalle de 50°C à 60°C.

7. Procédé suivant l'une quelconque des revendications 3 à 6, dans lequel l'étape de trimérisation et/ou de co-dimérisation (a) est mise en oeuvre à une pression suffisante pour maintenir l'éthylène et le 1-butène en phase liquide.

8. Procédé suivant la revendication 7, dans lequel la pression est comprise dans l'intervalle de 0,2 à 30 atm.

9. Procédé suivant l'une quelconque des revendications 3 à 8, dans lequel le catalyseur utilisé dans l'étape (a) est obtenu en faisant réagir un mélange d'un titanate d'alkyle et d'un éther avec un composé d'alkyl-aluminium.

10. Procédé suivant l'une quelconque des revendications 3 à 9, dans lequel l'étape de séparation (b) est mise en oeuvre dans une colonne d'adsorption ou en distillant ledit produit hydrocarboné pour le séparer des constituants de catalyseur pratiquement non volatils.

11. Procédé suivant la revendication 4, dans lequel l'étape consistant à séparer le produit en C₆ comprenant du 2-éthyl-1-butène du produit hydrocarboné est effectuée dans un dispositif de fractionnement.

12. Procédé suivant la revendication 11, dans lequel le dispositif de fractionnement comprend une première colonne qui est capable de séparer l'éthylène n'ayant pas réagi du 1-butène et des oléfines plus lourdes et une seconde colonne qui est capable de séparer le 1-butène d'un produit en C₆ et des produits oléfiniques plus lourds.

13. Procédé suivant la revendication 11 ou 12, dans lequel le dispositif de fractionnement comprend une première colonne qui est capable de séparer l'éthylène n'ayant pas réagi du 1-butène et des oléfines plus lourdes, une deuxième colonne qui est capable de séparer le 1-butène des oléfines en C₆ et oléfines plus lourdes et une troisième colonne qui est capable de séparer un produit en C₆ des oléfines en C₈ et oléfines plus lourdes.

14. Procédé suivant l'une quelconque des revendications 3 à 13, dans lequel l'étape d'éthérification (c) comprend les étapes consistant :
(i) à amener ledit produit hydrocarboné et au moins un alcool à un réacteur pour produire un mélange d'éthérification ; et
(ii) à provoquer la réaction catalytique dudit mélange d'éthérification dans ledit réacteur contenant un catalyseur d'éthérification dans des conditions de réaction qui favorisent la formation d'un courant contenant un éther à partir dudit produit hydrocarboné et dudit alcool.

15. Procédé suivant la revendication 14, dans lequel le catalyseur d'éthérification est un catalyseur consistant en une résine acide choisie dans le groupe consistant en : des catalyseurs consistant en résines échangeuses de cations et des catalyseurs macroréticulaires consistant en résines échangeuses de cations du type acide sulfonique.

16. Procédé suivant l'une quelconque des revendications 3 à 15, dans lequel l'étape d'éthérification (d) comprend l'introduction du courant contenant un éther dans une colonne de distillation pour séparer le produit consistant en éther dudit courant contenant un éther.

17. Procédé suivant la revendication 14, dans lequel l'alcool est le méthanol et le produit consistant en un éther est le 3-méthoxy-3-méthylpentane.

18. Procédé suivant la revendication 17, dans lequel l'étape de décomposition (e) permet de décomposer le 3-méthoxy-3-méthylpentane pour former au moins du 3-méthyl-2-pentène et du méthanol.

19. Procédé suivant l'une quelconque des revendications 3 à 18, dans lequel l'étape de séparation (f) comprend l'extraction avec de l'eau pour éliminer le ou les alcools du courant de 3-méthyl-2-pentène, et la distillation du 3-méthyl-2-pentène appauvri en alcool à partir dudit courant de 3-méthyl-2-pentène pour former ledit produit consistant en 3-méthyl-2-pentène.
